(19) Europäisches Patentamt
European Patent Office
Office européen des brevets

(11) Numéro de publication: **0 157 712**
**A1**

(12) # DEMANDE DE BREVET EUROPEEN

(21) Numéro de dépôt: **85420046.6**

(22) Date de dépôt: **13.03.85**

(51) Int. Cl.[4]: **C 07 D 213/32**, A 01 N 43/40

(30) Priorité: **15.03.84 FR 8404235**

(43) Date de publication de la demande: **09.10.85 Bulletin 85/41**

(84) Etats contractants désignés: **AT BE CH DE FR GB IT LI LU NL SE**

(71) Demandeur: **RHONE-POULENC AGROCHIMIE, 14-20, rue Pierre Baizet, F-69009 Lyon (FR)**

(72) Inventeur: **Place, Pierre, 173 Rue de la Mure, F-69300 Charly Vernaison (FR)**
Inventeur: **Anding, Claude, Domaine de la Source No 2, F-69630 Chaponost (FR)**
Inventeur: **Debourge, Jean-Claude, 14 Avenue des Frères Lumières, F-69410 Champagne Au Mont D'or (FR)**

(74) Mandataire: **Chaumette, Michel et al, RHONE-POULENC AGROCHIMIE BP 9163-09, F-69263 Lyon Cedex 1 (FR)**

(54) **Arylthio-pyridinyl-alcanols.**

(57) Dérivés d'arylthio-pyridinyl-alcanol répondant à la formule:

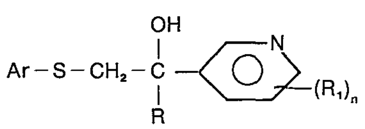

(I)

dans laquelle:

Ar représente un radical aryle éventuellement substitué,

R représente un atome d'hydrogène ou un radical alkyle éventuellement substitué, aryle éventuellement substitué ou aralkyle éventuellement substitué,

$R_1$ représente un radical alkyle et n est égal à 0, 1, 2, 3 ou 4.

EP 0 157 712 A1

La présente invention concerne de nouveaux arylthio-pyridinyl-alcanols, la préparation de ces produits ainsi que leur application pour la protection des végétaux, spécialement dans le cadre de la lutte contre les champignons parasites.

Un but de l'invention est la réalisation de produits à usage phytosanitaire notamment fongicide, pourvus de groupes pyridine-3 et sulfure substitué, actifs sur champignons du type Botrytis. Un autre but de l'invention est de fournir des fongicides ayant un large spectre d'activité. Il a maintenant été trouvé que ces buts pouvaient être atteints grâce aux produits de l'invention. Ceux-ci sont caractérisés en ce qu'ils répondent à la formule (I) :

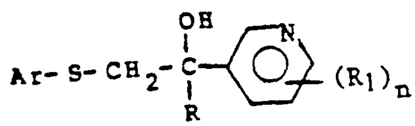

(I)

dans laquelle :

Ar représente un radical aryle éventuellement substitué,

R représente l'atome d'hydrogène ou un radical alkyle éventuellement substitué de préférence ramifié, aryle éventuellement substitué ou aralkyle éventuellement substitué.

$R_1$ représente un radical alkyle, linéaire ou ramifié,

n est un nombre entier égal à 0, 1, 2, 3 ou 4 étant entendu que lorsque n est supérieur à 1, les substituants $R_1$ peuvent être identiques ou différents.

Avantageusement, Ar représente un radical aryle comportant de 6 à 10 atomes de carbone (par exemple le radical phényle ou un radical naphtyle), éventuellement substitué par un ou plusieurs substituants identiques ou différents.

Les substituants de ce radical aryle peuvent être choisis parmi :

- les atomes d'halogène (de préférence chlore, fluor ou brome),
- les radicaux alkyle comportant de 1 à 12 atomes de carbone (par exemple méthyle, éthyle, isobutyle, etc.) éventuellement mono ou polyhalogénés (par exemple trifluorométhyle), alcényle et alcynyle comportant de 3 à 12 atomes de carbone (par exemple allyle, propargyle), alkoxy et alkylthio comportant de 1 à 6 atomes de carbone (par exemple méthoxy, méthylthio, éthoxy, isopropoxy...) éventuellement mono ou polyhalogénés (par exemple trifluorométhoxy, trifluorométhylthio) ; cyano; nitro ; phényle éventuellement substitué (par exemple phényle mono ou polyhalogéné) ; benzyle éventuellement substitué (par exemple halogéné) ; phénoxy éventuellement substitué ( par exemple halogéné) ; amino éventuellement substitué, (par exemple amino substitué par un à deux radicaux choisis parmi les radicaux alkyle comportant de 1 à 6 atomes de carbone et alcanoyle comportant de 2 à 6 atomes de carbone).

Avantageusement, R représente l'atome d'hydrogène ou un radical alkyle comportant de 1 à 10 atomes de carbone (de préférence un radical alkyle ramifié comportant de 3 à 5 atomes de carbone tel que isopropyle, butyle secondaire, tertio-butyle) ou un radical phényle ou benzyle, ces radicaux phényle et benzyle étant éventuellement substitués (par exemple par un ou plusieurs atomes d'halogène ou par un ou plusieurs radicaux alkyle comportant chacun de 1 à 6 atomes de carbone et eux-mêmes éventuellement substitués).

Avantageusement, $R_1$ représente un radical alkyle comportant de 1 à 6 atomes de carbone (par exemple méthyle, éthyle, isopropyle, t-butyle etc...) et n est égal à 0, 1 ou 2 ; de préférence n = 0.

Les composés selon l'invention peuvent exister sous une ou plusieurs formes d'isomères optiques. L'invention concerne donc aussi bien ces isomères optiques que leurs mélanges racémiques.

Dans le cas où il existe deux carbones asymétriques, les composés selon l'invention peuvent exister sous forme de diastéréoisomères qui sont également compris dans le cadre de la présente invention.

L'invention concerne également les formes salifiées des composés selon l'invention et plus spécialement les chlorhydrates, sulfates, oxalates.

La présente invention concerne aussi un procédé de préparation des composés selon l'invention.

Selon ce procédé, les composés de formule (I) peuvent être préparés par réaction d'un thiolate de formule (II) :

$$Ar\text{-}S\text{-}alc \qquad (II)$$

dans laquelle Ar a la même signification que dans la formule (I) et alc est un atome de métal alcalin, de préférence le sodium ou le potassium, avec une pyridine à groupe époxy de formule (III) :

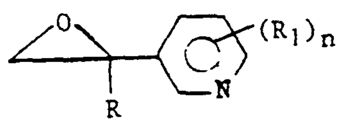

(III)

dans laquelle R, $R_1$ et n ont la même signification que dans la formule (I).

Le composé de formule (II) peut être préparé in situ ou non à partir d'un thiol aromatique de formule (IV) :

$$Ar\text{-}SH \qquad (IV)$$

dans laquelle Ar a la même signification que dans la formule (II), par action d'agents alcalins, par exemple des hydroxydes, carbonates, ou hydrures de métaux alcalins.

Les composés de formule (III) peuvent être préparés selon le procédé décrit dans J. Chem. Soc. Perkin Trans. J. 1982 (2) 587 à 590 par réaction d'un sel de diméthyl-oxo-sulfonium ou diméthylsulfonium en présence d'une base, sur une cétone de formule (V) :

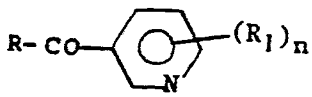 (V)

dans laquelle R, $R_1$ et n ont la même signification que dans la formule (III).

La réaction entre les composés de formule (II) et (III) s'effectue généralement entre -20 et +120°C de préférence entre 20 et 80°C. La durée de réaction peut varier dans de larges limites, par exemple 1 à 50 h ou plus souvent entre 1 et 5 h.

Les composés de formule (II) et (III) sont avantageusement mis en oeuvre stoechiométriquement mais peuvent aussi être mis en oeuvre dans des quantités s'écartant de la stoechiométrie, par exemple jusqu'à 100% en mole d'excés de thiolate de formule (II).

On opère avantageusement dans un solvant polaire.

Comme solvants susceptibles d'être utilisés on peut citer les solvants polaires aprotiques tels que le diméthylformamide (DMF), le diméthylsulfoxyde (DMSO) ; les nitriles tels que l'acétonitrile ; les solvants hydroxylés tels que les alcools liquides à température ambiante, notamment l'éthanol.

En fin de réaction le composé de formule (I) peut être isolé par tout moyen connu en soi par exemple par évaporation du milieu réactionnel puis extraction à l'aide d'un solvant non miscible à l'eau tel que par exemple des esters (acétate d'éthyle ou autres), des hydrocarbures halogénés (chloroforme, dichlorométhane).

Les cétones de formule (V) peuvent être préparées selon l'un ou l'autre des procédés connus décrits dans J. Am. Chem. Soc. 1951 - 73 - 469, Synthesis 1980 - p.1009, Org. Synth. Coll. Vol. IV 1963 p. 88, et plus particulièrement par hydrolyse des alpha-pyridyl, alpha-morpholino acétonitriles de formule (VI) :

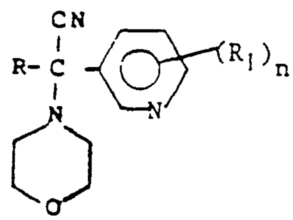

(VI)

dans laquelle R, $R_1$ et n ont la même signification que dans la formule (V), en opérant selon le procédé décrit par J. Org. Chem. 1979 - 44 (25) p. 4597.

Les composés de formule (VI) peuvent être ootenus par action de l'halogénure de formule (VII) :

R-hal (VII)

dans laquelle R a la même signification que dans la formule (VI) et hal représente un atome d'halogène, sur le composé de formule (VIiI) :

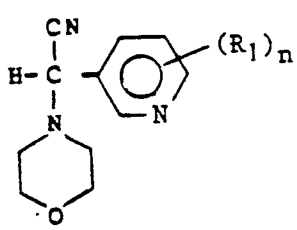

(VIII)

dans laquelle $R_1$ et n ont la même signification que précédemment, en opérant selon le procédé décrit dans J. Org. Chem. 1972 37 (26) p. 4465-4466).

Le composé selon la formule (V), pour lequel R représente le radical tertio-butyle et n est égal à zéro, peut avantageusement être préparé par méthylation du composé selon la formule (V) pour lequel R représente le radical isopropyle (par exemple par action de $ICH_3$ dans le DMF en présence de NaH). Ce composé peut aussi être préparé par oxydation de l'alcool de formule (IX) :

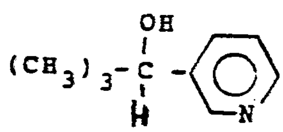

(IX)

en opérant selon la méthode décrite dans le brevet américain n° 4098908.

L'alcool de formule (IX) est avantageusement préparé par réaction du dérivé magnésien du chlorure de tertio-butyle dans un solvant éthéré comme l'éther éthylique ou le THF, avec la pyridine carboxaldéhyde-3.

Les exemples suivants donnés à titre non limitatif illustrent l'invention et montrent comment elle peut être mise en oeuvre.

Les exemples 1 à 63 et le tableau (I) illustrent la préparation des composés selon l'invention.

Les exemples 64 à 67 et le tableau (II) illustrent les propriétés fongicides des composés selon l'invention.

Dans les exemples 64 à 66 les pulvérisations de solutions ou suspensions de matières actives sont effectuées dans des conditions telles que la pulvérisation d'une solution ou suspension de concentration égale à 1 g/l corresponde en moyenne à l'application d'environ 2 microgrammes de matière active par $cm^2$ de feuille de la plante.

Dans ces exemples on considère qu'un produit exerce une protection totale vis-à-vis d'une maladie fongique lorsque la protection est d'au moins 95 % ; la protection est considérée comme bonne lorsqu'elle est d'au moins 80 %

(mais inférieure à 95 %), comme assez bonne lorsqu'elle est d'au moins 70 % (mais inférieure à 80 %), comme moyenne lorsqu'elle est d'au moins 50 % (mais inférieure à 70 %).

Dans le présent exposé les pourcentages sont, sauf indication contraire et sauf ceux concernant les rendements, des pourcentages pondéraux. Dans le cas où les pourcentages sont exprimés par rapport à la stoechiométrie, il s'agit de pourcentages molaires. En ce qui concerne les concentrations, certaines d'entre elles sont exprimées en ppm (partie par million) ce qui correspond à des mg/l.

Exemple 1 : Préparation du diméthyl-3,3 (méthyl-4' phénylthio)-1 (pyridin-3-yl)-2 butanol-2 :

A une solution de 4,38 g de méthyl-4 thiophénate de sodium (0,03 mole) dans 40 ml de DMF, on ajoute en 2 mn, à 20°C, une solution de 2,75 g (0,016 mole) de diméthyl-3,3 époxy-1,2 pyridin-3-yl)-2 butane, de formule :

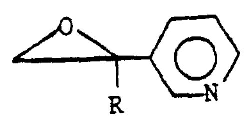

dans 40 ml de DMF.

On chauffe à 40°C pendant une heure. On coule ensuite le mélange réactionnel dans 200 ml d'eau froide et on extrait par 3 fois 50 ml d'acétate d'éthyle. On lave deux fois la phase organique à l'eau puis on concentre le solvant sous vide.

Après recristallisation dans l'hexane du solide obtenu, on obtient 2,6 g du composé cherché, de formule :

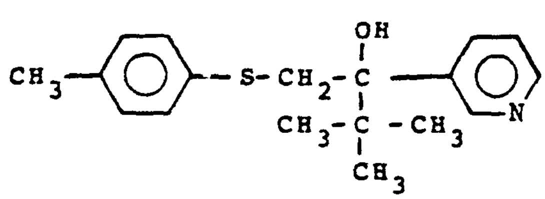

sous la forme d'un solide blanc fondant à 67°C (Rendement 57 %).

La structure de ce composé a été confirmée par Résonance Magnétique Nucléaire (RMN). Le spectre réalisé dans le chloroforme deutéré, avec comme référence interne le tétraméthylsilane est le suivant :

- un singulet à 0,95 ppm (9H), un singulet à 2,29 ppm (3H), un doublet à 3,33 ppm (1H), un doublet à 4,44 ppm (1H), un massif à 7,10 ppm (5H), un massif à 7,70 ppm (1H), un massif à 8,46 ppm (1H), un doublet à 8,64 ppm (1H).

Exemples 2 à 63

En opérant selon la méthode de l'exemple n° 1 à partir des matières premières appropriées, les composés n° 2 à 63 ci-après ont été préparés :

n° 2 : (bromo-4 phénylthio)-2 (pyridin-3 yl)-1 éthanol,
n° 3 : (pyridin-3 yl)-1 (trifluorométhyl-4 phénylthio)-2 éthanol,
n° 4 : (chloro-4 phénylthio)-1 (pyridin-3 yl)-2 propanol-2,
n° 5 : (pyridin-3 yl)-2 (trifluorométhyl-4 phénylthio)-1 propanol-2,
n° 6 : (bromo-4 phénylthio)-1 (pyridin-3 yl)-2 propanol-2,
n° 7 : (méthyl-4 phénylthio)-1 (pyridin-3 yl)-2 propanol-2,
n° 8 : (dichloro-2,4 phénylthio)-1 (pyridin-3 yl)-2 propanol-2,
n° 9 : (béta-naphtylthio)-1 (pyridin-3 yl)-2 propanol-2,
n° 10 : (béta-naphtylthio)-2 phényl-1 (pyridin-3 yl)-1 éthanol,
n° 11 : (chloro-4 phénylthio)-2 phényl-1 (pyridin-3 yl)-1 éthanol,
n° 12 : phényl-1 (pyridin-3 yl)-1 (trifluorométhyl-4 phénylthio)-2 éthanol,
n° 13 : (bromo-4 phénylthio)-2 phényl-1 (pyridin-3 yl)-1 éthanol,
n° 14 : (chloro-4 phényl)-1 (béta-naphtylthio)-2 (pyridin-3 yl)-1 éthanol,

n° 15 : (chloro-4 phénylthio)-2 (chloro-4 phényl)-1 (pyridin-3 yl)-1 éthanol,

n° 16 : (bromo-4 phénylthio)-2 (chloro-4 phényl)-1 (pyridin-3 yl)-1 éthanol,

n° 17 : (chloro-4 phényl)-1 (méthyl-4 phénylthio)-2 (pyridin-3 yl)-1 éthanol,

n° 18 : (bromo-4 phényl)-1 (chloro-4 phénylthio)-2 (pyridin-3 yl)-1 éthanol,

n° 19 : (bromo-4 phényl)-1 (méthyl-4 phénylthio)-2 (pyridin-3 yl)-1 éthanol,

n° 20 : (bromo-4 phényl)-1 (bromo-4 phénylthio)-2 (pyridin-3 yl)-1 éthanol,

n° 21 : (bromo-4 phényl)-1 phénylthio-2 (pyridin-3 yl)-1 éthanol,

n° 22 : (bromo-4 phényl)-1 (chloro-3 méthyl-4 phénylthio)-2-(pyridin-3 yl)-1 éthanol,

n° 23 : (bromo-4 phénylthio)-1 méthyl-3 (pyridin-3 yl)-2 butanol-2,

n° 24 : méthyl-3 (pyridin-3 yl)-2 (trifluorométhyl-4 phénylthio)-1 butanol-2,

n° 25 : (chloro-4 phénylthio)-1 méthyl-3 (pyridin-3 yl)-2 butanol-2,

n° 26 : (bromo-4 phénylthio)-1 méthyl-4 (pyridin-3 yl)-2 pentanol-2,

n° 27 : (chloro-4 phénylthio)-1 méthyl-4 (pyridin-3 yl)-2 pentanol-2,

n°28 : méthyl-4 (pyridin-3 yl)-2 (trifluorométhyl-4 phénylthio)-1 pentanol-2,

n° 29 : (chloro-4 phénylthio)-2 (pyridin-3 yl)-1 éthanol,

n° 30 : (chloro-4 phénylthio)-1 diméthyl-3,3 (pyridin-3 yl)-2 butanol-2,

n° 31 : (bromo-4 phénylthio)-1 diméthyl-3,3 (pyridin-3 yl)-2 butanol-2,

n° 32 : (dichloro-3,5 phénylthio)-1 diméthyl-3,3 (pyridin-3 yl)-2 butanol-2,

n° 33 : diméthyl-3,3 (fluoro-4 phénylthio)-1 (pyridin-3 yl)-2 butanol-2,

n° 34 : (chloro-4 méthyl-2 phénylthio)-1 diméthyl-3,3 (pyridin-3 yl)-2 butanol-2,

n° 35 : diméthyl-3,3 béta-naphtylthio-1 (pyridin-3 yl)-2 butanol-2,

n° 36 : (chloro-4 phénylthio)-1 méthyl-3 (pyridin-3 yl)-2 pentanol-2,

n° 37 : (bromo-4 phénylthio)-1 méthyl-3 (pyridin-3 yl)-2 pentanol-2,

n° 38 : (méthyl-4 phénylthio)-1 méthyl-3 (pyridin-3 yl)-2 pentanol-2,

n° 39 : (chloro-4 méthyl-3 phénylthio)-1 méthyl-3 (pyridin-3 yl)-2 pentanol-2,

n° 40 : (méthoxy-4 phénylthio)-1 méthyl-3 (pyridin-3 yl)-2 pentanol-2,

n° 41 : (fluoro-4 phénylthio)-1 méthyl-3 (pyridin-3 yl)-2 pentanol-2,

n° 42 : (n-dodécyl-4 phénylthio)-1 méthyl-3 (pyridin-3 yl)-2 pentanol-2,

n° 43 : méthyl-3 (n-nonyl-4 phénylthio)-1 (pyridin-3 yl)-2 pentanol-2,

n° 44 : méthyl-3 (pyridin-3 yl)-2 (trifluorométhyl-4 phénylthio)-1 pentanol-2,

n° 45 : méthyl-3 (pyridin-3 yl)-2 (2,4,5-$Cl_3$ phénylthio)-1 pentanol-2,

n° 46 : méthyl-3 (béta-naphtylthio)-1 (pyridin-3 yl)-2 pentanol-2,

n° 47 : diméthyl-3,3 (n-dodécyl-4 phénylthio)-1 (pyridin-3 yl)-2 butanol-2,

n° 48 : diméthyl-3,3 (n-nonyl-4 phénylthio)-1 (pyridin-3 yl)-2 butanol-2,

n° 49 : (dichloro-2,4 phénylthio)-1 diméthyl-3,3 (pyridin-3 yl)-2 butanol-2,

n° 50 : diméthyl-3,3 (béta-naphtylthio)-1 (pyridin-3 yl)-2 pentanol-2,

n° 51 : diméthyl-3,3 (fluoro-4 phénylthio)-1 (pyridin yl)-2 pentanol-2,

n° 52 : diméthyl-3,3 (n-dodécyl-4 phénylthio)-1 (pyridin-3 yl)-2 pentanol-2,

n° 53 : diméthyl-3,3 (n-nonyl-4 phénylthio)-1 (pyridin-3 yl)-2 pentanol-2,

n° 54 : (bromo-4 phénylthio)-1 diméthyl-3,3 (pyridin-3 yl)-2 pentanol-2,

n° 55 : (chloro-4 phénylthio)-1 diméthyl-3,3 (pyridin-3 yl)-2 pentanol-2,

n° 56 : diméthyl-3,3 (méthyl-4 phénylthio)-1 (pyridin-3 yl)-2 pentanol-2,

n° 57 : diméthyl-3,3 (méthoxy-4 phénylthio)-1 (pyridin-3 yl)-2 pentanol-2,

n° 58 : (chloro-4 phénylthio)-1 méthyl-3 (pyridin-3 yl)-2 hexanol-2,

n° 59 : (bromo-4 phénylthio)-1 méthyl-3 (pyridin-3 yl)-2 hexanol-2,

n° 60 : méthyl-3 (pyridin-3 yl)-2 (trifluorométhyl-4 phénylthio)-1 hexanol-2,

n° 61 : (bromo-4 phénylthio)-1 méthyl-3 (pyridin-3 yl)-2 nonanol-2,

n° 62 : (méthoxy-4 phénylthio)-1 méthyl-3 (pyridin-3 yl)-2 nonanol-2,

n° 63 : (béta-naphtylthio)-1 méthyl-3 (pyridin-3 yl)-2 nonanol-2.

Les formules et caractéristiques physicochimiques de ces composés sont indiquées dans le tableau I, situé à la fin de la description. La structure de ces composés a été confirmée par RMN.

Dans le cas des exemples n° 2 à 4, 6, 30 à 35, 44 à 46, 49, 54, 55 les rendements indiqués dans le tableau I concernent les produits obtenus par recristallisation dans l'hexane, selon la méthode de l'exemple 1. Dans le cas des composés n° 5, 7 à 29 et 36 à 43, 47, 48, 50 à 53, 56 à 63

ces rendements concernent les produits obtenus par chromatographie sur colonne de silice, avec comme éluant un mélange d'hexane et d'acétate d'éthyle.

Les indications figurant dans la colonne "delta" sont les valeurs des déplacements chimiques, en ppm des protons portés par le carbone situé en alpha du groupe Ar-S-, mesurés en utilisant comme référence interne le TMS (Tétraméthylsilane), à l'exception des exemples n° 3 à 8, 11, 45 et 58 à 60 pour lesquels on a utilisé comme référence interne l'HMDS (Héxaméthyldisiloxane).

Exemple 64 :

Test in vivo sur Erysiphe graminis sur orge (oïdium de l'orge)

On prépare par broyage fin une émulsion aqueuse de la matière active à tester ayant la composition suivante :

- matière active à tester 40 mg
- Tween 80 (agent tensioactif constitué d'un oléate de dérivé polyoxyéthylèné du sorbitan) 0,4 ml
- eau 40 ml.

Cette émulsion aqueuse est ensuite diluée par de l'eau pour obtenir la concentration désirée.

De l'orge, en godets, semée dans un mélange tourbe/pouzzolane, est traitée au stade 10 cm de hauteur par pulvérisation d'une émulsion aqueuse de concentration indiquée ci-après. L'essai est répété deux fois. Au bout de 24 heures, on saupoudre les plants d'orge avec des spores d'Erysiphe graminis, le saupoudrage étant effectué à l'aide de plants malades.

La lecture se fait 10 jours après la contamination.

Dans ces conditions, on observe une bonne protection avec les composés n° 1, 11, 15, 16, 45, à la dose de 330 ppm (parties par million). On obtient une bonne protection avec le composé n° 11 à la dose de 110 ppm.

Exemple 65 :

Test in vivo sur "Puccinia recondita" responsable de la rouille du blé

Du blé, en godets, semé dans un mélange tourbe/pouzzolane est traité au stade 10 [illegible] r pulvérisation avec des émulsions aqueuses de même composition que celle décrite à l'exemple 64 et à diverses concentrations du composé à tester. L'essai est répété deux fois avec chaque concentration.

Au bout de 24 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait le 15ème jour après la contamination par comparaison avec le témoin non traité.

Dans ces conditions, on observe une assez bonne protection avec les composés n° 10, 11, 15, 36, 63, utilisés à la dose de 330 ppm et une bonne protection avec les composés n° 37, 58, 59, 60, 61, utilisés à la même dose.

Exemple 66 :

Test sur Botrytis cinerea sur tomate :

Des tomates cultivées en serre (variété Marmande) agées de 60 à 75 jours sont traitées par pulvérisation avec des émulsions aqueuses de même composition que celle décrite à l'exemple 65 et à diverses concentrations du composé à tester. L'essai est répété deux fois avec chaque concentration.

Après 24 heures, les feuilles sont coupées et mises dans 2 boîtes de Pétri (diamètre 11cm) dont le fond a été préalablement garni d'un disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (80.000 unités/$cm^3$).

Le contrôle est fait 4 jours après la contamination par comparaison avec un témoin non traité.

Dans ces conditions on observe une protection totale avec les composés n° 36, 37, 38, 44, utilisés à la dose de 110 ppm.

Une bonne protection est obtenue avec les composés n° 12, 13, 16, 31, 40, 46, 50, 57, 58, 59, 60, à la dose de 330 ppm.

Exemple 67 :

Tests in vitro sur champignons des semences et champignons du sol

On étudie l'action des composés selon l'invention sur les champignons suivants responsables des maladies secondaires des céréales :

| | |
|---|---|
| Cercosporella herpotrichoïdes | (CERC) |
| Helminthosporium gramineum | (HELM G) |
| Pyrenophorae avenae | (PYRE) |
| Septoria nodorum | (SEPT N) |
| Helminthosporium teres | (HELM T) |
| Fusarium roseum | (FUS ROS) |
| Fusarium nivale | (FUS NIV) |
| Fusarium culmorum | (FUS CULM) |
| Rhizoctonia cerealis | (RHIZ C) |

Les indications figurant entre parenthèses seront utilisées pour représenter ces champignons dans le tableau (II), situé à la fin de la description.

Pour chaque essai, on opère de la manière suivante: dans des tubes à essai, on place 10 ml de milieu nutritif (pomme de terre + dextrose + agar-agar) puis on bouche

chaque tube et on le stérilise 20 mn à 120°C. Les tubes sont ensuite placés dans un bain-marie maintenu à 60°C. Ensuite, à l'aide d'une pipette, on injecte dans chaque tube une quantité déterminée d'une solution acétonique à 1% du composé à tester, de façon à obtenir dans le milieu une concentration déterminée de ce composé. Après homogénéisation, le milieu en surfusion contenu dans chaque tube est coulé aseptiquement dans une boîte de Petri de diamètre égal à 5 cm.

Au bout de 24 heures, chaque boîte est ensemencée par dépôt d'un fragment de mycelium (diamètre environ 8 mm) provenant d'une culture du champignon considéré.

On prend comme témoin une boîte analogue à la précédente, mais dont le milieu nutritif ne contient pas de matière active. Les boîtes sont conservées pendant 2 à 10 jours (selon le champignon testé) à 22°C et on compare alors la croissance du champignon dans les boîtes contenant la matière active à tester, à celle du même champignon dans la boîte utilisée comme témoin.

On détermine ainsi pour chaque composé testé la dose entraînant l'inhibition quasi totale (90-100 %) de la croissance du champignon.

Les composés qui entraînent l'inhibition quasi totale de la croissance des champignons à 10 ppm sont rapportées dans le tableau (II), situé à la fin de la description, dans lequel les abréviations ont la signification indiquée plus haut.

Les expérimentations décrites dans les exemples 64 à 67 illustrent clairement les bonnes propriétés fongicides des composés selon l'invention.

Ces composés peuvent donc être utilisés pour la lutte tant préventive que curative contre les champignons, notamment de type basidiomycètes, ascomycètes, adelomycetes ou fungi-imperfecti, en particulier les rouilles, oïdium, fusarioses, helminthosporioses, septorioses, rhizoctones

des végétaux et des plantes en général et en particulier des céréales telles que le blé, l'orge, le seigle, l'avoine et leurs hybrides et aussi le riz et le maïs.

Les produits de l'invention sont spécialement intéressants par leur spectre large au niveau des maladies des céréales (oïdium, rouille, piétin verse, helmintnosporioses, septorioses et en particulier les fusarioses difficiles à combattre).

Ils présentent également un grand intérêt en plus de leur large spectre par leur bonne activité sur Botrytis et de ce fait peuvent être appliqués sur des cultures aussi variées que la vigne, les cultures maraichères et l'arboriculture.

Ils s'appliquent avantageusement à des doses de 0,05 à 5kg/ha, de préférence de 0,1 à 2kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, contiennent comme matière active un composé selon l'invention tel que décrit précédemment en association avec les supports solides ou liquides, acceptables en agriculture et éventuellement les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les

composés selon l'invention peuvent être associés à tous les additifs solides ou liquides correspondant aux techniques habituelles de la mise en formulation.

Les doses d'emploi peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$ % à 95 % (en poids).

Selon ce qui a déjà été dit les composés selon l'invention sont généralement associés à des supports et éventuellement des agents tensioactifs. Leur teneur en agent tensioactif est généralement comprise entre 0 % et 20 % en poids.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les

émulsions ou solutions prêtes à l'application contenant, quant à elles, 0,01 à 20 % de matière active. En plus du solvant, les concentrés émulsionnables peuvent contenir, quand c'est nécessaire, un co-solvant approprié et de 2 à 20 % d'additifs appropriés, comme des stabilisants, des agents tensioactifs, des agents de pénétration, des inhibiteurs de corrosion, des colorants, des adhésifs. A titre d'exemple, voici la composition de quelques concentrés émulsionnables :

| | | |
|---|---|---|
| - matière active | | 400 g/l |
| - dodécylbenzène sulfonate alcalin | | 24 g/l |
| - nonylphénol oxyéthylé à 10 molécules d'oxyde d'éthylène | | 16 g/l |
| - cyclohexanone | | 200 g/l |
| - solvant aromatique | q.s.p | 1 litre |

Selon une autre formule de concentré émulsionnable, on utilise :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme

support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus de la matière active et du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

- matière active 400 g
- lignosulfonate de sodium 50 g
- dibutylnaphtalène sulfonate de sodium 10 g
- silice 540 g

Une autre composition de poudre à pulvériser à 25 % utilise les constituants suivants :

- matière active 250 g
- lignosulfonate de calcium 45 g
- mélange équipondéral de craie de Champagne et d'hydroxyéthylcellulose 19 g
- dibutylnaphtalène sulfonate de sodium 15 g
- silice 195 g
- craie de Champagne 195 g
- kaolin 281 g

Une autre composition de poudre à pulvériser à 25 % utilise les constituants suivants :

- matière active 250 g
- isooctylphénoxy-polyoxyéthylène-éthanol 25 g
- mélange équipondéral de craie de Champagne et d'hydroxyéthylcellulose 17 g
- aluminosilicate de sodium 543 g
- kieselguhr 165 g

Une autre composition de poudre à pulvériser à 10 % utilise les constituants suivants :

- matière active 100 g
- mélange de sels de sodium de sulfates d'acides gras saturés 30 g
- produit de condensation d'acide naphtalène sulfonique et de formaldéhyde 50 g
- kaolin 820 g

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont

la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

- matière active 50 g
- épichlorhydrine 2,5 g
- éther de cétyle et de polyglycol 2,5 g
- polyéthylène glycol 35 g
- kaolin (granulométrie : 0,3 à 0,8 mm) 910 g.

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin

avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

L'invention concerne de plus un procédé pour lutter contre les maladies fongiques des végétaux. Ce procédé est caractérisé en ce qu'il consiste à appliquer sur ces végétaux une quantité efficace d'une composition contenant comme matière active un composé selon la formule (I). Par quantité "efficace" on entend une quantité suffisante pour combattre la maladie fongique, sans endommager les végétaux traités. Les doses d'utilisation peuvent varier dans de larges limites selon le champignon à combattre, le type de culture, les conditions climatiques et le composé utilisé. Comme indiqué plus haut, ces doses sont avantageusement comprises entre 0,05 et 5 kg/ha.

TABLEAU I

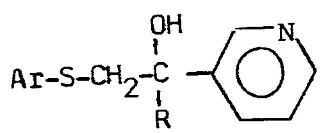

| Exemple | Ar | R | Rendement (%) | Point de fusion | delta ppm |
|---|---|---|---|---|---|
| 1 | $p-CH_3-C_6H_4-$ | $-C(CH_3)_3$ | 57 % | 67°C | |
| 2 | $p-Br-C_6H_4-$ | H | 70 % | 89°C | 3.20 |
| 3 | $p-CF_3-C_6H_4-$ | H | 31 % | 73°C | 3.24 |
| 4 | $p-Cl-C_6H_4-$ | $-CH_3$ | 59 % | 75°C | 3.39 - 3.25 |
| 5 | $p-CF_3-C_6H_4-$ | $-CH_3$ | 37 % | huile | 3.45 - 3.33 |
| 6 | $p-Br-C_6H_4-$ | $-CH_3$ | 93 % | 57°C | 3.34 - 3.24 |
| 7 | $p-CH_3-C_6H_4-$ | $-CH_3$ | 58 % | huile | 3.34 - 3.23 |
| 8 | $o,p-Cl_2-C_6H_3-$ | $-CH_3$ | 46 % | huile | 3.29 - 3.23 |
| 9 | ß-naphtyle | $-CH_3$ | 100 % | huile | 3.41 - 3.25 |
| 10 | ß-naphtyle | $-C_6H_5$ | 57 % | huile | 4.00 - 3.85 |
| 11 | $p-Cl-C_6H_4-$ | $-C_6H_5$ | 35 % | huile | 3.78 - 3.68 |

TABLEAU I (Suite)

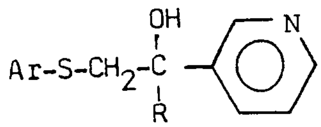

| Exemple | Ar | R | Rendement (%) | Point de fusion | delta ppm |
|---|---|---|---|---|---|
| 12 | $p-CF_3-C_6H_4-$ | $-C_6H_5$ | 27 % | huile | 4.00 - 3.77 |
| 13 | $p-Br-C_6H_4-$ | $-C_6H_5$ | 62 % | huile | 3.90 - 3.67 |
| 14 | ß-naphtyle | $p-Cl-C_6H_4-$ | 33 % | huile | 4.00 - 3.77 |
| 15 | $p-Cl-C_6H_4-$ | $p-Cl-C_6H_4-$ | 37 % | huile | 3.90 - 3.67 |
| 16 | $p-Br-C_6H_4-$ | $p-Cl-C_6H_4-$ | 67 % | huile | 3.87 - 3.67 |
| 17 | $p-CH_3-C_6H_4-$ | $p-Cl-C_6H_4-$ | 14 % | huile | 3.82 - 3.67 |
| 18 | $p-Cl-C_6H_4-$ | $p-Br-C_6H_4-$ | 59 % | huile | 3.82 - 3.65 |
| 19 | $p-CH_3-C_6H_4-$ | $p-Br-C_6H_4-$ | 32 % | huile | 3.85 - 3.65 |
| 20 | $p-Br-C_6H_4-$ | $p-Br-C_6H_4-$ | 31 % | huile | 3.85 - 3.62 |
| 21 | $C_6H_5-$ | $p-Br-C_6H_4-$ | 53 % | huile | 3.90 - 3.70 |
| 22 | $m-Cl,p-CH_3-C_6H_3-$ | $p-Br-C_6H_4-$ | 44 % | huile | 3.75 - 3.62 |

TABLEAU I (Suite)

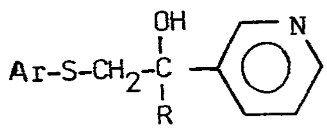

| Exemple | Ar | R | Rendement (%) | Point de fusion | delta ppm |
|---|---|---|---|---|---|
| 23 | $p\text{-}Br\text{-}C_6H_4\text{-}$ | $-CH(CH_3)_2$ | 47 % | huile | 3.70 - 3.35 |
| 24 | $p\text{-}CF_3\text{-}C_6H_4\text{-}$ | $-CH(CH_3)_2$ | 49 % | huile | 3.72 - 3.38 |
| 25 | $p\text{-}Cl\text{-}C_6H_4\text{-}$ | $-CH(CH_3)_2$ | 62 % | huile | 3.70 - 3.37 |
| 26 | $p\text{-}Br\text{-}C_6H_4\text{-}$ | $-CH_2\text{-}CH(CH_3)_2$ | 56 % | huile | 4.00 - 3.38 |
| 27 | $p\text{-}Cl\text{-}C_6H_4\text{-}$ | $-CH_2\text{-}CH(CH_3)_2$ | 47 % | huile | 4.02 - 3.40 |
| 28 | $p\text{-}CF_3\text{-}C_6H_4\text{-}$ | $-CH_2\text{-}CH(CH_3)_2$ | 52 % | huile | 3.98 - 3.35 |
| 29 | $p\text{-}Cl\text{-}C_6H_4\text{-}$ | -H | 66 % | huile | 3.20 |
| 30 | $p\text{-}Cl\text{-}C_6H_4\text{-}$ | $-C\ (CH_3)_3$ | 67 % | 105°C | 4.04 - 3.36 |
| 31 | $p\text{-}Br\text{-}C_6H_4\text{-}$ | $-C(CH_3)_3$ | 71 % | 113°C | 4.00 - 3.37 |
| 32 | $3,5\text{-}Cl_2\text{-}C_6H_3\text{-}$ | $-C(CH_3)_3$ | 24 % | 116°C | 3.69 - 3.10 |
| 33 | $p\text{-}F\text{-}C_6H_4\text{-}$ | $-C(CH_3)_3$ | 48 % | 68°C | 4.03 - 3.30 |

TABLEAU I (Suite)

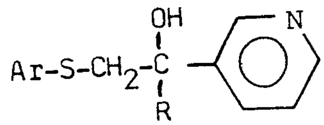

| Exemple | Ar | R | Rendement (%) | Point de fusion | delta ppm |
|---|---|---|---|---|---|
| 34 | o-$CH_3$,p-Cl-$C_6H_3$ | -$C(CH_3)_3$ | 43 % | 136°C | 3.96 - 3.30 |
| 35 | ß-naphtyle | -$C(CH_3)_3$ | 52 % | 74°C | 4.12 - 3.48 |
| 36 | p-Cl-$C_6H_4$- | -$CH(CH_3)$-$C_2H_5$ | 66 % | huile | 3.85 - 3.40 |
| 37 | p-Br-$C_6H_4$- | -$CH(CH_3)C_2H_5$ | 66 % | huile | 3.95 - 3.42 |
| 38 | p-$CH_3$-$C_6H_4$- | -$CH(CH_3)$-$C_2H_5$ | 70 % | huile | 3.71 - 3.77 |
| 39 | o-$CH_3$,p-Cl-$C_6H_3$- | -$CH(CH_3)$-$C_2H_5$ | 60 % | huile | 3.80 - 3.38 |
| 40 | p-$CH_3O$-$C_6H_4$ | -$CH(CH_3)$-$C_2H_5$ | 74,5 % | huile | 3.68 - 3.32<br>3.68 - 3.31 |
| 41 | p-F-$C_6H_4$ | -$CH(CH_3)$-$C_2H_5$ | 91,5 % | huile | 3.67 - 3.38<br>3.65 - 3.38 |
| 42 | p-n$C_{12}H_{25}$-$C_6H_4$ | -$CH(CH_3)$-$C_2H_5$ | 96,5 % | huile | 3.48 - 3.81<br>3.46 - 3.81 |

TABLEAU I (Suite)

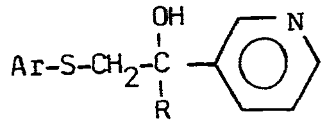

| Exemple | Ar | R | Rendement (%) | Point de fusion | delta ppm |
|---|---|---|---|---|---|
| 43 | p-$nC_9H_{19}$-$C_6H_7$ | -$CH(CH_3)$-$C_2H_5$ | 94 % | huile | 3.41 - 3.72<br>3.39 - 3.72 |
| 44 | p-$CF_3$-$C_6H_4$ | -$CH(CH_3)$-$C_2H_5$ | 11 % | 87°C | 3.51 - 3.75<br>3.49 - 3.75 |
| 45 | 2,4,5-$Cl_3$-$C_6H_2$ | -$CH(CH_3)$-$C_2H_5$ | 15 % | 116°C | 3.65 - 3.33<br>3.63 - 3.31 |
| 46 | β-naphtyle | -$CH(CH_3)$-$C_2H_5$ | 70 % | 105°C | 3.51 - 3.77<br>3.49 - 3.77 |
| 47 | p-$nC_{12}H_{25}$-$C_6H_4$ | -$C(CH_3)_3$ | 53,5 % | huile | 3.35 - 4.05 |
| 48 | p-$nC_9H_{19}$-$C_6H_4$ | -$C(CH_3)_3$ | 46 % | huile | 3.34 - 4.04 |
| 49 | 2,4$Cl_2$-$C_6H_3$ | -$C(CH_3)_3$ | 17 % | 131°C | 3.29 - 4.05 |
| 50 | β-naphtyle | -$C(CH_3)_2$-$C_2H_5$ | 30 % | huile | 3.47 - 4.13 |
| 51 | p-F-$C_6H_4$ | -$C(CH_3)_2$-$C_2H_5$ | 32 % | huile | 3.32 - 4.02 |

TABLEAU I (Suite)

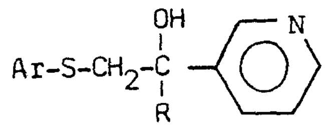

| Exemple | Ar | R | Rendement (%) | Point de fusion | delta ppm |
|---|---|---|---|---|---|
| 52 | p-n$C_{12}H_{25}$-$C_6H_4$ | -C$(CH_3)_2$-$C_2H_5$ | 30 % | huile | 3.36 - 4.08 |
| 53 | p-$C_9H_{19}$-$C_6H_4$ | -C$(CH_3)_2$-$C_2H_5$ | 25 % | huile | 3.36 - 4.06 |
| 54 | p-Br-$C_6H_4$ | -C$(CH_3)_2$-$C_2H_5$ | 20 % | 119°C | 3.36 - 4.07 |
| 55 | p-Cl-$C_6H_4$ | -C$(CH_3)_2$-$C_2H_5$ | 17 % | 108°C | 3.34 - 4.05 |
| 56 | p-$CH_3$-$C_6H_4$ | -C$(CH_3)_2$-$C_2H_5$ | 15 % | huile | 3.32 - 4.02 |
| 57 | p-$CH_3O$-$C_6H_4$ | -C$(CH_3)_2$-$C_2H_5$ | 30,5 % | huile | 3.25 - 4.01 |
| 58 | p-Cl-$C_6H_4$ | -CH$(CH_3)$n$C_3H_7$ | 52,5 % | huile | 3.63 - 3.36<br>3.60 - 3.34 |
| 59 | p-Br-$C_6H_4$ | -CH$(CH_3)$n$C_3H_7$ | 47,5 % | huile | 3.59 - 3.37<br>3.58 - 3.36 |
| 60 | p-$CF_3$-$C_6H_4$ | -CH$(CH_3)$n$C_3H_7$ | 24,5 % | huile | 3.68 - 3.46<br>3.65 - 3.45 |

TABLEAU I (Suite)

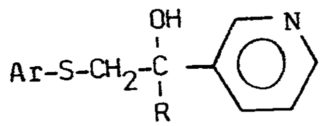

| Exemple | Ar | R | Rendement (%) | Point de fusion | delta ppm |
|---|---|---|---|---|---|
| 61 | p-Br-$C_6H_4$ | $-CH(CH_3)nC_6H_{13}$ | 29,5 % | huile | 3.82 - 3.42<br>3.80 - 3.41 |
| 62 | p-$CH_3O$-$C_6H_4$ | $-CH(CH_3)nC_6H_{13}$ | 27 % | huile | 3.72 - 3.32<br>3.71 - 3.31 |
| 63 | ß-naphtyle | $-CH(CH_3)nC_6H_{13}$ | 10 % | huile | 3.82 - 3.53<br>3.80 - 3.52 |

TABLEAU II

| CHAMPIGNONS | COMPOSES INHIBANT LA CROISSANCE DES CHAMPIGNONS A LA DOSE DE 10 PPM |
|---|---|
| CERC | 2-23-26-27-31-36-37-38-39-40-41-44-49-50-51-54-55-56-57-58-59-60 |
| HELM-G | 23-26-27-30-31-37-38-39-44-46-49-54-55-56-57-58-59-60 |
| PYRE | 23-26-27-30-31-36-37-38-39-40-44-46-49-51-54-55-56-57-58-59-60 |
| HELM-T | 10-13-23-26-27-30-31-36-37-38-39-40-41-44-46-49-50-51-54-55-56-57-58-59-60 |
| SEPT-N | 1-17-23-26-27-30-31-36-37-38-39-40-41-44-46-49-51-54-55-56-57-58-59-60 |
| FUS-ROS | 1-36-37-38-46 |
| FUS-NIV | 12 |
| FUS-CULM | 1-12-23-26-36-38-40-41-44-49-50-51-54-55-56-57-58-59-60 |
| RHIZ-C | 1-30-56-60 |

REVENDICATIONS

POUR LES ETATS CONTRACTANTS : BE, CH, DE, FR, GB, IT, LI, LU, NL, SE

1) Dérivé d'arylthio-pyridinyl-alcanol caractérisé en ce qu'il répond à la formule générale (I) :

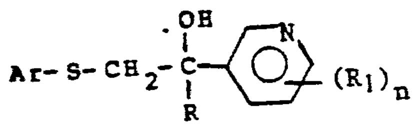

(I)

dans laquelle :

Ar représente un radical aryle éventuellement substitué,

R représente un atome d'hydrogène ou un radical alkyle éventuellement substitué, aryle éventuellement substitué ou aralkyle éventuellement substitué,

$R_1$ représente un radical alkyle,

n est égal à 0, 1, 2, 3 ou 4 étant entendu que lorsque n est supérieur à 1 les substituants $R_1$ peuvent être soit identiques soit différents et formes salifiées de ce composé.

2) Composé selon la revendication 1 caractérisé en ce que Ar représente un radical aryle comportant de 6 à 10 atomes de carbone, éventuellement substitué par un ou plusieurs atomes ou radicaux, identiques ou différents choisis parmi les atomes d'halogènes, les radicaux alkyle comportant de 1 à 12 atomes de carbone, éventuellement mono ou polyhalogénés, alcényle et alcynyle comportant de 3 à 12 atomes de carbone, alkoxy et alkylthio comportant de 1 à 6 atomes de carbone éventuellement mono ou polyhalogénés, cyano, nitro, phényle éventuellement substitué, benzyle éventuellement substitué, phénoxy éventuellement substitué et amino éventuellement substitué,

R représente l'atome d'hydrogène ou un radical alkyle comportant de 1 à 10 atomes de carbone, phényle éventuellement substitué ou benzyle éventuellement substitué,

$R_1$ représente un radical alkyle comportant de 1 à 6 atomes de carbone,

et n est égal à 0, 1 ou 2.

3) Composé selon l'une des revendications 1 et 2 caractérisé en ce que R représente un radical alkyle ramifié comportant de 3 à 5 atomes de carbone.

4) Composition utilisable pour la protection des végétaux contre les maladies fongiques caractérisée en ce qu'elle contient comme matière active un composé selon l'une des revendications 1 à 3.

5) Composition selon la revendication 4 caractérisée en ce que, en plus de la matière active, elle contient un ou plusieurs supports acceptables en agriculture et/ou un ou plusieurs agents tensio-actifs acceptables en agriculture.

6) Composition selon la revendication 5 caractérisée en ce qu'elle contient en poids de 0,5 à 95 % de matière active.

7) Procédé de préparation d'un composé selon la revendication 1 caractérisé en ce qu'on fait réagir un thiolate de formule (II)

Ar-S-alc

dans laquelle Ar a la même signification que dans la revendication 1 et alc représente un atome de métal alcalin, sur la pyridine à groupe époxy, de formule (III)

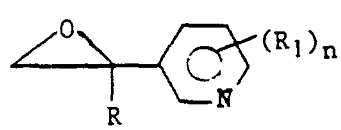

(III)

dans laquelle R, $R_1$ et n ont la même signification que dans la revendication 1.

0157712



8) Procédé selon la revendication 7 caractérisé en ce qu'on opère à une température comprise entre moins 20°C et plus 120°C, en présence d'un solvant.

9) Procédé pour lutter contre les maladies fongiques des végétaux, caractérisé en ce qu'il consiste à appliquer sur les végétaux une quantité efficace d'un composé selon l'une des revendication 1 à 3.

REVENDICATIONS POUR L'ETAT CONTRACTANT : AT

1) Composition utilisable pour la protection des végétaux contre les maladies fongiques, caractérisée en ce qu'elle contient comme matière active un dérivé d'arylthio-pyridinyl-alcanol répondant à la formule générale (I):

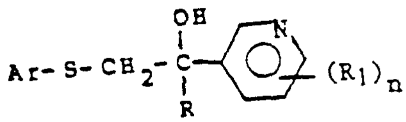

(I)

dans laquelle :

Ar représente un radical aryle éventuellement substitué,

R représente un atome d'hydrogène ou un radical alkyle éventuellement substitué, aryle éventuellement substitué ou aralkyle éventuellement substitué,

$R_1$ représente un radical alkyle,

n est égal à 0, 1, 2, 3 ou 4 étant entendu que lorsque n est supérieur à 1 les substituants $R_1$ peuvent être soit identiques soit différents ou une forme salifiée de ce composé.

2) Composition selon la revendication 1 caractérisée en ce qu'elle contient comme matière active un composé répondant à la formule (I) dans laquelle :

Ar représente un radical aryle comportant de 6 à 10 atomes de carbone, éventuellement substitué par un ou plusieurs atomes ou radicaux, identiques ou différents choisis parmi les atomes d'halogènes, les radicaux alkyle comportant de 1 à 12 atomes de carbone, éventuellement mono ou polyhalogénés, alcényle et alcynyle comportant de 3 à 12 atomes de carbone, alkoxy et alkylthio comportant de 1 à 6

atomes de carbone éventuellement mono ou polyhalogénés, cyano, nitro, phényle éventuellement substitué, benzyle éventuellement substitué, phénoxy éventuellement substitué et amino éventuellement substitué,

R représente l'atome d'hydrogène ou un radical alkyle comportant de 1 à 10 atomes de carbone, phényle éventuellement substitué ou benzyle éventuellement substitué,

$R_1$ représente un radical alkyle comportant de 1 à 6 atomes de carbone,

et n est égal à 0, 1 ou 2.

3) Composition selon l'une des revendications 1 et 2 caractérisée en ce que R représente un radical alkyle ramifié comportant de 3 à 5 atomes de carbone.

4) Composition selon l'une des revendications 1 à 3 caractérisée en ce que, en plus de la matière active, elle contient un ou plusieurs supports acceptables en agriculture et/ou un ou plusieurs agents tensio-actifs acceptables en agriculture.

5) Composition selon la revendication 4 caractérisée en ce qu'elle contient en poids de 0,5 à 95 % de matière active.

6) Procédé de préparation d'un composé répondant à la formule générale (I)

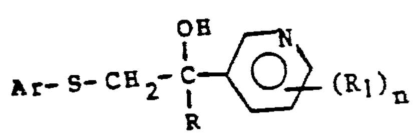

(I)

dans laquelle Ar, R, $R_1$ et n ont la même signification que dans la revendication 1, caractérisé en ce qu'on fait réagir un thiolate de formule (II)

Ar-S-alc

dans laquelle Ar a la même signification que précédemment et alc représente un atome de métal alcalin, sur la pyridine à groupe époxy, de formule (III)

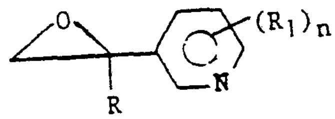

(III)

dans laquelle R, $R_1$ et n ont la même signification que précédemment.

7) Procédé selon la revendication 6 caractérisé en ce qu'on opère à une température comprise entre moins 20°C et plus 120°C, en présence d'un solvant.

8) Procédé pour lutter contre les maladies fongiques des végétaux, caractérisé en ce qu'il consiste à appliquer sur les végétaux une quantité efficace d'une composition selon l'une des revendication 1 à 5.

Office européen des brevets

# RAPPORT DE RECHERCHE EUROPEENNE

0157712

Numero de la demande

EP 85 42 0046

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | EP-A-0 001 399 (BAYER AG)<br>* En entier *<br>--- | 1,4 | C 07 D 213/32<br>A 01 N 43/40 |
| A | EP-A-0 074 018 (F. HOFFMANN-LA ROCHE & CO.)<br>* Revendications *<br>--- | 1,4,7 | |
| A | US-A-3 869 456 (E. LILLY & CO.)<br>* Colonnes 1-3 *<br>--- | 1,4 | |
| A | FR-A-2 130 555 (E. LILLY & CO.)<br>* Revendications; page 2, en bas *<br>----- | 1,4 | |
| | | | DOMAINES TECHNIQUES RECHERCHES (Int. Cl.4) |
| | | | C 07 D 213/00 |

Le présent rapport de recherche a été établi pour toutes les revendications

| Lieu de la recherche | Date d'achèvement de la recherche | Examinateur |
|---|---|---|
| LA HAYE | 18-06-1985 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82